# EUROPEAN PATENT APPLICATION

(11) **EP 4 302 819 A1**
(43) Date of publication of application: **10.01.2024**
(21) Application number: 22183509.3
(22) Date of filing: 07.07.2022
(51) Int. Cl.: A61N 1/05, A61N 1/08

(54) **A PADDLE LEAD**

(71) Applicant: BIOTRONIK SE & Co. KG, 12359 Berlin (DE)
(72) Inventor: van Arx, Jeffrey A., Lake Oswego, 97035 (US); Stotts, Larry, Fulshear, 77441 (US)
(74) Representative: Biotronik Corporate Services SE

(57) **Abstract**

The invention relates to a paddle lead (1) for implantation in a patient's epidural space, the paddle lead (1) comprising: an lead body (2); a paddle structure (3) electrically coupled to a distal portion of the lead body (2) and arranged distally of the lead body (2), wherein the paddle structure (3) comprises: a core substrate (9) made from a liquid crystal polymer; an electrically insulating cover layer (10) surrounding the core substrate (9) at least section-wise; a plurality of therapeutic electrode poles (4) arranged on a surface of the paddle structure (3) not covered by the electrically insulating cover layer (10); at least one energy dissipating electrode pole (5) arranged on a surface of the paddle structure (3) not covered by the electrically insulating cover layer (10) nor by the plurality of therapeutic electrode poles (4); at least one capacitor (6) electrically interconnecting at least one of the plurality of therapeutic electrode poles (4) with the at least one energy dissipating electrode pole (5); wherein the plurality of therapeutic electrode poles (4) and the at least one energy dissipating electrode pole (5) are electrically interconnected with each other only by the at least one capacitor (6).

## Description

### TECHNICAL FIELD

The present disclosure generally relates to a paddle lead for implantation in a patient's epidural space, as well as to a method for manufacturing such a paddle lead.

### BACKGROUND

Paddle leads are regularly used for spinal cord stimulation (SCS) and are placed for this purpose in the epidural space close to the spinal cord.

The term "paddle lead" originates from the flattened, paddle-like shape of these electrodes. Paddle leads carry an arrangement of multiple evenly or unevenly distributed electrode poles (also referred to as contact elements) on their surface for emission of electrical signals into and/or reception of electrical signals from surrounding tissue.

Paddle leads are often implanted in a surgical manner close to the height of the final site of action. For this purpose, parts of a vertebral body in this region are removed to provide necessary space for implantation.

However, it is also known to implant paddle leads in a minimally invasive way like percutaneous electrodes. To allow such minimally invasive implantation, it is necessary to compress the paddle lead to a delivery state in which the outer diameter of the paddle lead is reduced so that the paddle lead can be guided through an implantation catheter used for minimally invasive implantation. Once positioned at its intended site of implantation, the paddle lead is released from the implantation catheter and transferred from its (collapsed) delivery state into its (expanded) implantation state.

When subjecting a patient, to whom a paddle lead has been implanted, to magnetic resonance imaging (MRI), the applied magnetic resonance (MR) radiofrequency (RF) field may lead to heating of the tissue near each electrode on the paddle lead Therefore, patients carrying an implanted paddle lead are often subjected to MRI examinations in which only a limited amount of energy is introduced into the patient's body. This, however, results in less sensitive MRI results.

Paddle leads for spinal cord stimulation (SCS) are more difficult to design for MR conditional safety than percutaneous SCS leads. This is primarily due to the MRI induced radiofrequency (RF) electrode heating hazard. In general, paddle SCS leads dissipate RF energy at the electrodes with significantly more heating than percutaneous SCS leads. There are two main reasons for this. First, the paddle lead electrodes are in general smaller area than percutaneous lead electrode area, and smaller electrodes heat more than larger electrodes when dissipating energy. Typical electrode area for a paddle lead is ~5 mm², while the electrode area for percutaneous leads are typically ∼25 mm². To a first order, heating is inversely proportional to electrode area, so based just on this, paddle leads can be expected to have several times the RF induced heating of a typical percutaneous lead. A second reason that paddle leads tend to heat more is that paddle leads only dissipate RF induced power in one hemisphere of tissue. This is because paddle leads are planar and surrounded by a large insulating surface (the paddle). In contrast, percutaneous electrodes are cylindrical and therefore dissipate RF induced power in 360 degrees of tissue. Since the paddle lead dissipates its energy in a smaller volume of tissue (one hemisphere as opposed to both hemispheres), it results in more tissue heating even if all other parameters (such as electrode area) were equal.

There is one paddle lead on the market that allows an MRI body scan in normal operating mode without power restrictions. This paddle lead comprises two lead bodies for connecting the paddle leads with an implantable pulse generator (IPG). Both lead bodies comprise a braided shield applied around the lead bodies. Due to this braided shield, a significant less amount of RF energy is introduced into the paddle lead during an MRI scan. However, the braided shield solution makes for a complicated lead body that adds cost and complexity to the lead body. It also results in a thicker and stiffer lead body.

US 2017/0080213 A1 describes an implantable neurostimulation lead comprising a lead body having a distal end and a proximal end, and a lumen extending along a longitudinal axis between the distal and proximal ends. A multi-layer coil is wound about the lumen and extends at least partially along a length of the lead body. The multi-layer coil includes a first winding formed with multiple winding turn segments wound about the lumen in a first direction about the longitudinal axis. The multi-layer coil further includes a second winding formed with multiple winding turn segments wound about the first winding in an opposite second direction about the longitudinal axis.

US 7,174,219 B2 describes an electrode body having a therapy electrode that is electrically coupled to a stimulation lead for delivering therapy to the patient. A floating electrode is configured to contact the patient's body tissue and has a surface area substantially larger than that of the therapy electrode. A filter is coupled between the therapy electrode and the floating electrode for diverting radiofrequency (RF) energy toward the floating electrode and away from the therapy electrode. The floating electrode comprises a conductive metal plate to enlarge its surface.

US 8,509,913 describes an energy management system that facilitates the transfer of high frequency energy induced on an implanted lead. This system includes an energy-dissipating surface associated with the implanted lead, a diversion circuit associated with the energy-dissipating surface, and at least one switch for diverting energy in the implanted lead through the diversion circuit to the energy-dissipating surface. In alternate configurations, the switch may be disposed between the implanted lead and the diversion circuit, or disposed so that it electrically opens the implanted lead when diverting energy through the diversion circuit to the energy-dissipating surface. The switch may comprise a single or multi-pole double or single throw switch. The diversion circuit may be either a high pass filter or a low pass filter.

### SUMMARY

It is an object of the present invention to provide a paddle lead that allows full power MRI examinations of a patient to whom the paddle lead is implanted and that can be easier manufactured than comparable paddle leads known from prior art.

This object is achieved with a paddle lead having the features explained in the following. Such a paddle lead serves for implantation in the patient's epidural space. The paddle lead comprises one or more lead bodies and a paddle structure electrically coupled to the distal end of the lead body. In this context, the paddle structure is arranged at the distal end of the lead body. The paddle structure comprises a core substrate that comprises or essentially consists of a liquid crystal polymer (LCP). According to prior art solutions, no such liquid crystal polymer is used for manufacturing a core substrate of a paddle lead. Rather, other materials are used for this purpose. However, a liquid crystal polymer allows a highly integrated design of the paddle lead and a particularly easy manufacturing process. A liquid crystal polymer is a multilayer flexible substrate material that can be structured by a photolithographic printing technique. It allows for all components, and routing to be printed on a single substrate which is then molded into the paddle insulator material (typically silicone) rather than all components and connecting wires being individually molded into the paddle insulating material. A liquid crystal polymer allows a very good integration of individual components into the core substrate of the paddle lead and guarantees an easy manufacturing in a very robust, reliable and reproducible manner. Liquid crystal polymer is biocompatible, does not degrade in the body environment, and it has very low moisture absorption and moisture penetration properties.

According to an embodiment of the present invention, the core substrate of the paddle structure comprises or essentially consists of LCP laminates which are stacked with deposited metal layers. Moreover, the core substrate of the paddle structure comprises or essentially consists of at least one of the following specific types of LCP: ULTRALAM 3850, Zenite, Vectra or Laperos.

The paddle structure further comprises an electrically insulating cover layer (typically made of silicone) surrounding the core substrate at least in sections. Furthermore, the paddle structure comprises a plurality of therapeutic electrode poles that is arranged on a surface of the paddle structure not being covered by the electrically insulating cover layer. Consequently, the therapeutic electrode poles can get into direct contact with the surrounding tissue after the paddle lead has been implanted into the patient's body. The therapeutic electrode poles are electrode poles for delivering therapy and/or for sensing action potentials from the tissue.

The paddle structure further comprises at least one energy dissipating electrode pole that is arranged on a part of the surface of the paddle structure that is also not covered by the electrically insulating cover layer. In addition, the energy dissipating electrode pole is arranged in a section of the surface of the paddle structure that is also not covered by the plurality of therapeutic electrode poles. Consequently, also the at least one energy dissipating electrode pole can get in direct contact with the surrounding tissue and does not interfere with the therapeutic electrode poles. The energy dissipating electrode pole is able to safely dissipate MR induced RF energy with minimal heating due to its large size (it has a significantly larger area than the summed area of the therapeutic electrode poles). Thus, heating at any single location is minimized and the risk of unintentional stimulation of the spinal cord is mainly minimized because the RF energy is spread out over a large electrode.

The paddle structure further comprises at least one capacitor that electrically interconnects at least one of the plurality of therapeutic electrode poles with the at least one energy dissipating electrode pole. In this context, the at least one capacitor is the only electrical connection between the plurality of therapeutic electrode poles and the at least one energy dissipating electrode pole. The at least one capacitor can also be denoted as bypass capacitor. The at least one bypass capacitor allows high frequency signals such as MR induced RF power to pass, but present a high impedance to low frequency SCS stimulating signals.

In an embodiment, the core substrate is at least partially surrounded by the electrically insulating cover layer, the plurality of therapeutic electrode poles, and the at least one energy dissipating electrode pole. In an embodiment, the core substrate is fully surrounded by the electrically insulating cover layer, the plurality of therapeutic electrode poles, and the at least one energy dissipating electrode pole. Thus, these three elements (the cover layer, the therapeutic electrode poles and the energy dissipating electrode pole) surrounds the core substrate such that there is no direct contact between the core substrate and the surrounding tissue in the implanted state of the paddle lead. Such an arrangement is particularly appropriate for protecting the core substrate against any influences from the surrounding tissue. The insulating cover layer is typically silicone, which also provide a soft interface between the paddle structure and the surrounding tissue, which minimize the risk of any mechanical trauma to the tissue.

In an embodiment, each of the plurality of therapeutic electrode poles is electrically connected with the at least one energy dissipating electrode pole via an individual capacitor. Thus, in this embodiment, the number of capacitors equals the number of therapeutic electrode poles, wherein some or all of the therapeutic electrode poles are electrically connected with the same energy dissipating electrode.

In an embodiment, the at least one capacitor is an integrated capacitor that is integrated into the core substrate. Such an integrated capacitor can be realized by electric traces provided within the core substrate and being spaced apart from each other by a defined distance, thus leading to capacitance between the individual wires. Since the liquid crystal polymer allows a very precise manufacturing of structures embedded within the core substrate, the manufacturing of such integrated capacitors is particularly easy. In an embodiment, these integrated capacitors consist of wide metal traces sandwiched between layers of a second set of wide medal traces. The dielectric for these integrated capacitors is the substrate material itself, with typically a single layer of the substrate material serving as the dielectric.

In an embodiment, the at least one capacitor is a discreet capacitor embedded into the core substrate. Also such an arrangement can be well realized by manufacturing the core substrate from a liquid crystal polymer, even though the scale of the paddle lead is somewhat increased when using discrete capacitors instead of integrated capacitors since discrete capacitors have a minimum space requirement being larger than the space requirement of integrated capacitors. Nonetheless, the liquid crystal polymer can be well applied around discrete capacitors, wherein the wiring necessary for electrically connecting the discrete capacitors can be integrated into the liquid crystal polymer.

In an embodiment, the paddle structure has a front side that is configured for facing the patient's spinal cord in the implanted state of the paddle structure. At the same time, the paddle structure has a back side opposite the front side and configured for facing away from the patient's spinal cord in the implanted state of the paddle lead. In this embodiment, both the plurality of therapeutic electrode poles and the at least one energy dissipating electrode are arranged on the front side, i.e., facing a patient's spinal cord in the implanted state of the paddle lead. Since the energy dissipating electrode pole has typically a significantly larger area than the sum of the areas of the plurality of therapeutic electrode poles (it is, e.g., at least 10 %, in particular at least 20 %, in particular at least 30 %, in particular at least 40 %, in particular at least 50 %, in particular at least 60 %, in particular at least 70 %, in particular at least 80 %, in particular at least 90 %, in particular at least 100 %, in particular at least 150 %, in particular at least 200 %. in particular at least 300 %, in particular at least 400 %, in particular at least 600 %, in particular at least 800 %, in particular at least 1000 %, in particular at least 1200 %, in particular at least 1400 % larger than the area of the plurality of therapeutic electrode poles), it does not significantly heat upon introduction of radiofrequency energy. Rather, it dissipates the MR-induced radiofrequency energy into the surrounding tissue. Due to this minimum heating, it is generally unproblematic if the energy dissipating electrode pole faces the spinal cord of the patient in the implanted state of the paddle lead.

In an embodiment, the at least one energy dissipating electrode pole is arranged in a central area between the plurality of therapeutic electrode poles. It may thus cover a full central area of the front side of the paddle structure. In another embodiment, the energy dissipating electrode pole is proximal, distal, or on one or both sides of the therapeutic electrode poles rather than in the center between the therapeutic electrode poles.

In an embodiment, the paddle structure has a front side that is configured for facing the patient's spinal cord in the implanted state of the paddle lead. At the same time, the paddle structure has a back side opposite the front side and being configured for facing away from a patient's spinal cord. In this embodiment, the plurality of therapeutic electrode poles is arranged on the front side to easily emit electric pulses to the patient's spinal cord. At the same time, the at least one energy dissipating electrode pole is arranged on the back side of the paddle structure. In doing so, the energy impact onto the spinal cord upon radiating radiofrequency onto the paddle lead is further reduced since this energy is dissipated by the energy dissipating electrode pole away from the patient's spinal cord. Thus, this embodiment further reduces the risk of any injuries of the spinal cord due to a radiofrequency irradiation, e.g., by an MRI examination of the patient.

As already explained above, using a thin film substrate rather than discrete wiring and discrete electrodes in the paddle results in a particularly thin design of the paddle substrate. In an embodiment, the core substrate has a thickness lying in a range of from 12,5µm to 20µm, in particular of from 20µm to 25µm, in particular of from 50 µm to 500 µm, in particular of from 75 µm to 450 µm, in particular of from 100 µm to 400 µm, in particular of from 200 µm to 300 µm. Liquid crystal polymer (LCP) is preferred over other biocompatible substrate material such as polyimide LCP has extremely low moisture absorption, which makes its properties much more stable in an medical device lead. Due to this reduced thickness of the paddle compared to the discrete paddle assemblies available today, the paddle lead adapts itself in a more physiological way to the anatomy of the patient to whom the paddle lead is implanted. paddle lead

In an embodiment, the at least one capacitor and the at least one energy dissipating electrode pole are arranged in the same plane of the core substrate. In addition, the at least one capacitor is covered by an additional layer of liquid crystal polymer. This results in an embedding of the capacitor within the liquid crystal polymer and a particularly small distance between the energy dissipating electrode pole and the capacitor(s). This, in turn, facilitates the establishment of an electric connection between the at least one capacitor and the at least one energy dissipating electrode pole and thus the overall manufacturing of the paddle lead. Since the liquid crystal polymer allows a layer-by-layer setup of the core substrate, it enables a particularly flexible manufacturing of the core substrate and the electric connections and components embedded within the core substrate to realize a transfer of energy away from the therapeutic electrode poles and towards the energy dissipating electrode pole.

In an embodiment, each of the plurality of therapeutic electrode poles is electrically connected with the at least one capacitor via one connecting trace per each of the plurality of therapeutic electrode poles. Typically, each of the plurality of therapeutic electrode poles is electrically interconnected with a single capacitor via an integrated trace on the substrate. In this context, the traces are arranged in either the same layer of the core substrate as the plurality of therapeutic electrode poles is arranged, or in different layers interconnected by vias. Furthermore, the printed traces on the LCP are covered by an additional layer of liquid crystal polymer. Also by this planar arrangement between the connecting traces and the therapeutic electrode poles, a particularly simple manufacturing of the paddle lead is made possible. Due to the additional layer of liquid crystal polymer covering the connecting traces, the traces are well protected against external influences so that they show a high durability.

In an embodiment, the at least one capacitor has a capacitance lying in a range of from 100 pF to 10 nF, in particular of from 200 pF to 9 nF, in particular of from 300 pF to 8 nF, in particular of from 400 pF to 7 nF, in particular of from 500 pF to 6 nF, in particular of from 600 pF to 5 nF, in particular of from 700 pF to 4 nF, in particular of from 800 nF to 3 nF, in particular of from 900 pF to 2 nF, in particular of from 950 pF to 1.5 nF. Capacitors having a capacitance in this range are much more appropriate for guiding radiofrequency energy away from the therapeutic electrode poles and towards the energy dissipating electrode pole than parasitic capacitors having a much lower capacitance. To give a specific example, a capacitance lying around a range of 1 nF is particularly appropriate for the present purposes.

At 64 MHz (the RF frequency for a 1.5 T MRI) and 128 MHz (the RF frequency for a 3.0 T MRI), a 1 nF bypass capacitance yields reactances of 2.5 Ω and 1.2 Ω respectively. These reactances are sufficiently small to shunt most of the RF energy away from the therapeutic electrode poles and into the large energy dissipating electrode pole. Note that although the bypass capacitor adds a few Ω impedance to the path to the large energy dissipating electrode pole, the much lower impedance of the large electrode pole ensures most high frequency current takes this path rather than through the smaller, and thus higher impedance-stimulating therapeutic electrodes poles. At SCS stimulation frequencies (up to 10 kHz), the InF bypass capacitance has a reactance of > 15 kΩ. This is sufficient to ensure that practically the entire SCS stimulation signal is blocked from the energy dissipating electrode pole.

In an embodiment, only a subset of the plurality of therapeutic electrode poles is electrically interconnected with the at least one energy dissipating electrode pole. In this context, the subset consists of those therapeutic electrode poles that are most prone to magnetic resonance energy heating during operation of the paddle lead. In a variant of this embodiment, the 2 to 8, in particular the 3 to 7, in particular the 4 to 6 therapeutic electrode poles that are most prone to magnetic resonance energy heating belong to the subset. Typically, each therapeutic electrode pole in the subset is connected via an individual capacitor (e.g., a discrete capacitor or an embedded capacitor) with the energy dissipating electrode pole. This guarantees a highly reliable energy guidance away from those therapeutic electrode poles that are prone to a significant heating upon an RF energy introduction into the patient's body.

In the following, some more physical background on electrode heating is given. As explained above, not all electrode poles on the paddle lead are prone to the same MR-induced heating. This is because heating is very sensitive to wire length, and the wire lengths connecting each electrode pole (at the distal end of the lead) to the medical device connector (at the proximal end of the lead) is not the same for each electrode. At the distal end of the lead, some electrodes are more distal than others, and at the proximal end, some connectors are more proximal than others. This results in wire lengths that differ by as much as ~8 cm. The reason that some wire lengths are more sensitive to heating than others is electrical resonance. Wire lengths that are near the electrical resonance length or a harmonic of the electrical resonance length of the MRI will pick up several times more RF radiated power than other lengths that are far from electrical resonance. There are other effects that can cause differences in heating between electrodes as well. For example, coupling between neighboring wires can cause some wires to have more RF heating than others. It is possible to analyze which electrodes are the most prone to heating at MR RF frequencies. In one embodiment, only the hottest electrodes are coupled to the energy dissipating electrode pole. For example, in one embodiment only the two hottest electrodes are coupled to the energy dissipating electrode pole. This minimizes the number of bypass capacitors that are needed. In an embodiment only a subset of the therapeutic electrode poles on the paddle (consisting of what would otherwise be the hottest electrodes) are connected to the energy dissipating electrode pole.

Integrated bypass capacitors having a capacitance in the order of 1 nF can be achieved by overlapping wide metal areas on the substrate. The required overlap area is calculated as follows: C=kε₀ A/d, where C is the target capacitance, k is the dielectric constant for substrate material (for LCP k = ~3.16), where d is layer thickness separating the metal traces (for typical LCP processes, d = 25 µm), ε₀ is the dielectric constant for free space (8.85 · 10⁻¹² F/m), and A is the required area. Solving for area yields a required area of ~5 cm² for a single 1-nF capacitor. A typical 16-electrode paddle is about 6 cm² in area (~ 6 cm by 1 cm). An integrated capacitor design with alternating layers of electrode conductors requires up to 2.5 cm² in area since the capacitor would be on both sides of a single layer. Therefore, it is reasonable in a 9-layer LCP substrate to fit 16 1-nF capacitors. Since in one embodiment only the hottest electrodes are coupled to the energy dissipating electrode pole, only two bypass capacitors are needed in this embodiment. In this embodiment, a 4-layer LCP substrate would be adequate to fit the capacitors plus the integrated electrodes.

In an embodiment, the at least one energy dissipating electrode pole comprises or essentially consists of platinum, gold, iridium, platinum iridium, iridium oxide, titanium nitride, and/or poly(3,4-ethylenedioxythiophene) (also referred to PEDOT). In an embodiment, the plurality of therapeutic electrode poles comprises or essentially consists of platinum, gold, iridium, platinum iridium, iridium oxide, titanium nitride, and/or poly(3,4-ethylenedioxythiophene). In an embodiment, the at least one energy dissipating electrode pole and the plurality of therapeutic electrode poles are made from the same material. This further facilitates the manufacturing of the paddle lead.

In an embodiment, the electrically insulating cover layer comprises or essentially consists of at least one of silicone, polyimide, polyester, and PEEK. Silicone is particularly appropriate as electrically insulating cover layer. A silicone coating results in a soft, durable, and biocompatible tissue interface.

In an aspect, the present invention relates to a method for manufacturing a paddle lead according to the preceding explanations. This method comprises the steps explained in the following.

In one method step, an lead body is provided. In another method step, a paddle structure is arranged distally of the lead body and is electrically coupled to a distal portion of the lead body. The paddle structure comprises a core substrate that comprises or essentially consists of a liquid crystal polymer (LCP).

The paddle structure further comprises an electrically insulating cover layer surrounding the core substrate at least in sections. Furthermore, the paddle structure comprises a plurality of therapeutic electrode poles that is arranged on a surface of the paddle structure not being covered by the electrically insulating cover layer.

The paddle structure further comprises at least one energy dissipating electrode pole that is arranged on a part of the surface of the paddle structure that is also not covered by the electrically insulating cover layer. In addition, the energy dissipating electrode pole is arranged in a section of the surface of the paddle structure that is also not covered by the plurality of therapeutic electrode poles.

The paddle structure further comprises at least one capacitor that electrically interconnects at least one of the plurality of therapeutic electrode poles with the at least one energy dissipating electrode pole. In this context, the at least one capacitor is the only electrical connection between the plurality of therapeutic electrode poles and the at least one energy dissipating electrode pole.

In an embodiment, the electrode pole material, as well as the metal traces for the capacitors and/or the wires can be deposited by sputtering, electroplating, evaporation, or other thin film deposition techniques.

In an embodiment, the electrically insulating cover layer is a silicone coating that is applied by dipping the paddle structure into liquid silicone, by overmolding the paddle structure, by compression molding, by applying calendared silicone sheeting, and/or another silicone coating technique. The electrode poles need to be exposed to tissue (i.e. not coated in silicone), which can be achieved by either masking the electrode poles during the coating process, or by etching the silicone over the electrodes after the coating process (e.g. laser etching of silicone, or reactive ion etching).

If discrete capacitors are used, and if they are too tall for embedding them directly into the core substrate, then another option is used according to an embodiment. In this embodiment, the discrete components are encapsulated with the core substrate material by placing a frame made of the core substrate material around the discrete capacitor, and then folding the flexible core substrate over the top. The frame is welded to the bottom core substrate and the top is folded over the core substrate lid by, for example, resistance welding which heats locally the LCP, melting the structures together.

Manufacturing those embodiments of the paddle lead, in which the energy dissipating electrode pole is located on the front side of the paddle structure, may be easier accomplished than manufacturing those embodiments of the paddle structure, in which the energy dissipating electrode pole is located on the back side of the paddle structure for a couple of reasons. First, it requires less masking of the silicone over molding. With this design, it is possible to have a single opening in the silicone over molding expose all therapeutic electrode poles and energy dissipating electrode pole. Even if separate openings in the silicone over modeling are desired, the embodiment with a front side energy dissipating electrode pole has the advantage of having all those openings on the same side, simplifying masking of the silicone layer. A second reason that a front side energy dissipating electrode pole is easier to manufacture is that all tissue-interfacing electrode poles are located on the same core substrate layer (the top). In contrast, the embodiment with the back side energy dissipating electrode pole requires two layers (top and bottom) with tissue-interfacing electrode poles.

The connection between the paddle structure and the distal end of the lead (or the wires inside the lead, such as MP35N cables with a silver core, that electrically connect the paddle structure to the proximal lead terminal) is, in an embodiment, formed by welding the wires to the paddle structure. This welding is, in an embodiment, laser welding, although other welding techniques can generally be used. In an embodiment, a welding stub of a weld-friendly material (e.g. platinum, gold, platinum iridium) is crimped to the end of the wires so that there are favorable metals for welding together at the weld stub to the paddle structure interface.

All embodiments of the paddle lead can be combined in any desired way and can be transferred either individually or in any arbitrary combination to the manufacturing method. Likewise, all embodiments of the manufacturing method can be combined in any desired way and can be transferred either individually or in any arbitrary combination to the paddle lead.

### DESCRIPTION OF THE DRAWINGS

Further details of aspects of the present invention will be explained in the following making reference to exemplary embodiments and accompanying Figures. In the Figures:
- Figure 1A: shows the front side of a first embodiment of a paddle lead;
- Figure 1B: shows the back side of the paddle lead of Figure 1A;
- Figure 2A: shows the front side of a second embodiment of a paddle lead;
- Figure 2B: shows the back side of the paddle lead of Figure 2A;
- Figure 3A: shows the front side of a third embodiment of a paddle lead;
- Figure 3B: shows the front side of a fourth embodiment of a paddle lead;
- Figure 4: shows a schematic cross-sectional view through a fifth embodiment of a paddle lead;
- Figure 5: shows a schematic cross-sectional view through a sixth embodiment of a paddle lead; and
- Figure 6: shows a schematic cross-sectional view through a seventh embodiment of a paddle lead.

### DETAILED DESCRIPTION

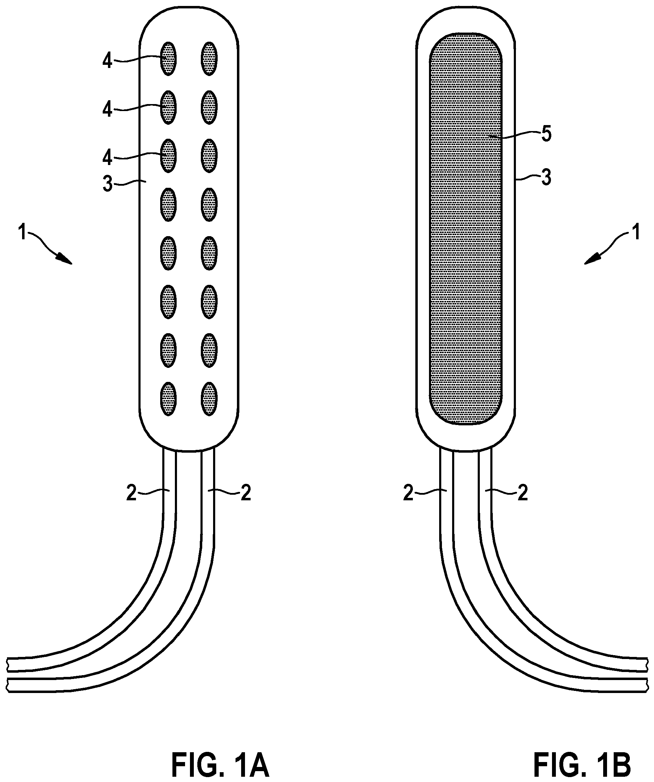

Figure 1A shows the front side of a paddle lead 1 that comprises two lead bodies 2 and a paddle structure 3 connected to the two lead bodies 2. The paddle structure 3 comprises 16 discrete therapeutic electrode poles 4, only some of which are marked with the respective numeral reference.

Each of the therapeutic electrode poles 4 is connected with an individual wire that is guided through one of the two lead bodies 2 to an implantable pulse generator comprising a sensing unit for sensing electric signals via the therapeutic electrode poles 4 and a stimulation unit for emitting stimulation pulses via the therapeutic electrode poles 4.

Figure 1B shows the back side of the same paddle lead 1. In this and in all following Figures, similar elements will be denoted with the same numeral reference.

The front side of the paddle lead 1 can also be denoted as spinal cord facing site, since it is intended to face a patient's spinal cord in the implanted state of the paddle lead 1. The back side of the paddle lead 1 can also be denoted as non-spinal cord facing surface, since it is intended to face away from a patient's spinal cord in the implanted state of the paddle lead 1.

The back side of the paddle lead 1 comprises a single large energy dissipating electrode pole 5 that covers most of the surface of the back side of the paddle lead 1. This energy dissipating electrode pole 5 is intended to guide radiofrequency energy and/or heat induced by such radiofrequency energy away from the patient's spinal cord in the implanted state of the paddle lead 1.

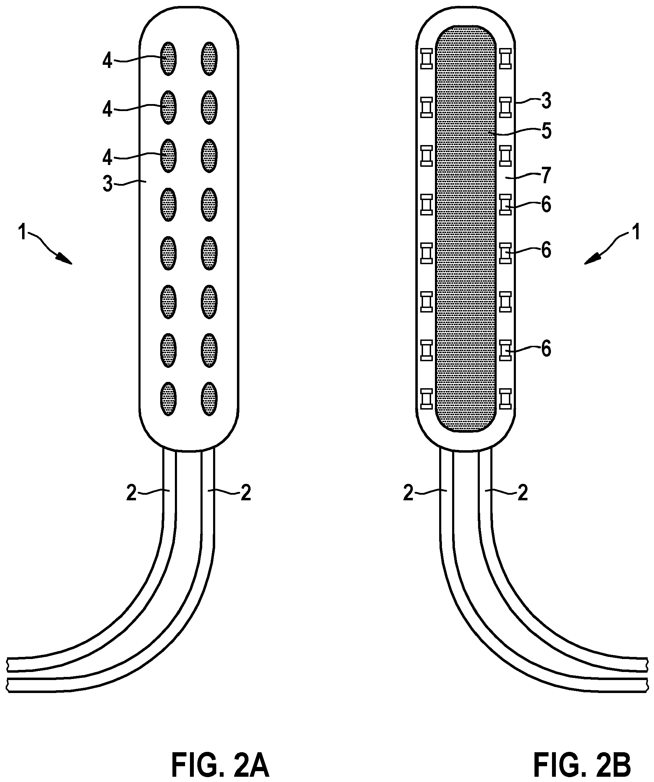

Figure 2A shows the front side of another embodiment of a paddle lead 1. This front side is identical to the front side of the paddle lead 1 shown in Figure 1A so that reference is made to the explanations given with respect to Figure 1A.

Figure 2B shows the back side of the paddle lead 1 of Figure 2A. This back side differs from the back side of the paddle lead 1 shown in Figure 1B. In particular, 16 discrete capacitors 6 are arranged in an edge region 7 of the back side of the paddle lead 1. Each of the discrete capacitors 6 is assigned to a single of the therapeutic electrode poles 4 (cf. Figure 2A). To be more precise, each of the therapeutic electrode poles 4 is connected via a single of the capacitors 6 with the energy dissipating electrode pole 5. In doing so, radiofrequency energy irradiated onto the paddle lead 1 will be safely kept away from the therapeutic electrode poles 4 and will be introduced into the energy dissipating electrode pole 5 from which it can be dissipated into the surrounding tissue.

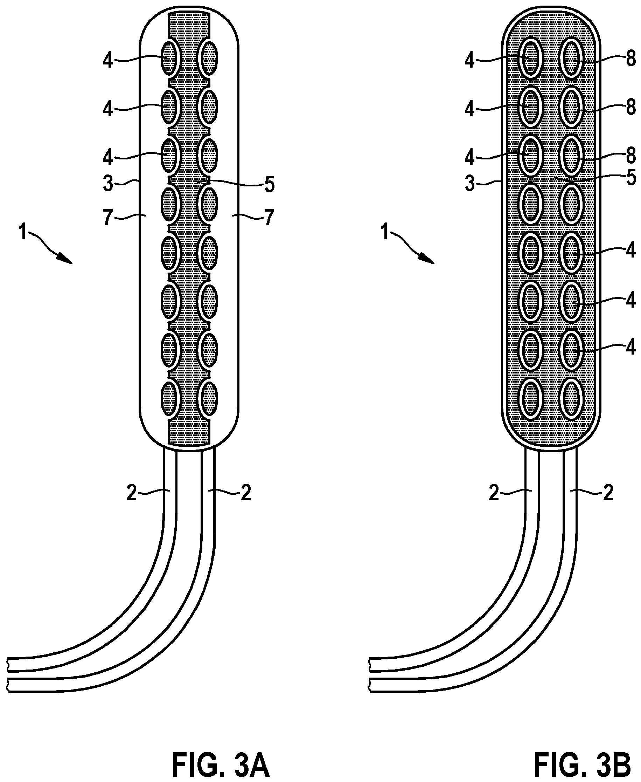

Figure 3A shows the front side of another embodiment of a paddle lead 1 in which both the therapeutic electrode poles 4 and the energy dissipating electrode pole 5 are arranged on the same side of the paddle lead 1. To be more precise, the energy dissipating electrode pole 5 is arranged in a central area between the individual therapeutic electrode poles 4. For this purpose, some geometric complexity has been added to the energy dissipating electrode pole 5 to maximize the size of the energy dissipating electrode pole 5. The larger the area of the energy dissipating electrode pole 5, the lower is the corresponding electrode impedance, and hence the more radiofrequency energy (radiofrequency current) it will shut away from the therapeutic electrode poles 4. To maximize its area, the energy dissipating electrode pole 5 comprises partial cutouts leaving space for the therapeutic electrode poles 4. The energy dissipating electrode pole 5 does not protrude into the lateral edge region 7 of the paddle structure 3, but is only arranged in a central region of the paddle structure 3.

Figure 3B shows the front side of another embodiment of a paddle lead 1. In this embodiment, the energy dissipating electrode pole 5 completely surrounds each of the therapeutic electrode poles 4, while leaving spaces 8 between the energy dissipating electrode pole 5 and each of the therapeutic electrode poles 4, thus avoiding a direct electric contact between the energy dissipating pole 5 and the therapeutic electrode poles 4. Thus, also in this embodiment, an electric contact between each of the therapeutic electrode poles 4 and the energy dissipating electrode pole 5 is only established via a plurality of capacitors (not shown in Figure 3B).

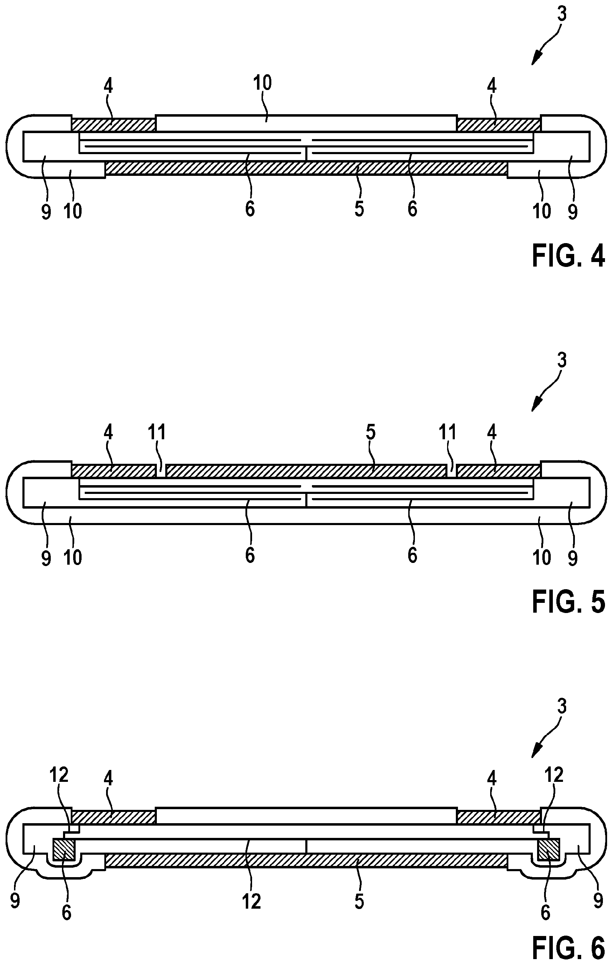

Figure 4 shows a cross-sectional view through a paddle structure 3 of another embodiment of a paddle lead. The paddle structure 3 comprises a core substrate 9 that is made from a liquid crystal polymer. A first and second capacitor 6 are integrated into the core substrate 9. Each of the capacitors 6 electrical interconnects one therapeutic electrode pole 4 with the same (and only) energy dissipating electrode pole 5. All surface regions of the core substrate 9 that are not covered by either the therapeutic electrode poles 4 or the energy dissipating electrode pole 5 are coated with a silicone layer 10, serving as electrically insulating cover layer.

The therapeutic electrode poles 4 are arranged on the front side of the paddle structure 3, whereas the energy dissipating electrode pole 5 is arranged on a back side of the paddle structure 3.

Figure 5 shows a cross-sectional view through the paddle structure 3 of another embodiment of a paddle lead. In this embodiment, both the therapeutic electrode poles 4 and the energy dissipating electrode pole 5 are arranged on the same side, namely on the front side of the paddle structure 3. While a silicone layer 10 covers most of the surface parts of the core structure 9 that are not covered by either of the therapeutic electrode poles 4 or the energy dissipating electrode pole 5, the silicone layer 10 does not cover all of these surface regions. Rather, there remain two small gaps 11 between the therapeutic electrode poles 4 and the energy dissipating electrode pole 5, which is arranged in the center between the therapeutic electrode poles 4. Due to these gaps 11, a direct electric contact between the therapeutic electrode poles 4 and the energy dissipating electrode pole 5 is avoided. Rather, an electrical contact between the therapeutic electrode poles 4 and the energy dissipating electrode pole 5 is still established only via the capacitors 6 embedded within the core substrate 9. The gaps 11 are due to the manufacturing process of the paddle structure 3. By allowing these gaps 11, only a single opening in the silicone layer 10 on the front side of the paddle structure 3 is necessary. Due to these gaps 11, a small part of the core substrate 9 is directly exposed to bodily fluids and tissue. Since the core substrate 9 is made from a liquid crystal polymer, i.e., an inert, biocompatible material, this direct contact does not negatively influence the electric properties of the paddle structure 3.

Figure 6 shows a cross-sectional view through a paddle structure 3 of another embodiment of a paddle lead. This embodiment generally resembles the embodiment shown in Figure 4. Thus, the therapeutic electrode poles 4 are located on the front side of the paddle structure 3, whereas the energy dissipating electrode pole 5 is located on a back side. The main difference between the embodiment shown in Figure 6 and the embodiment shown in Figure 4 is the use of discrete capacitors embedded within the core substrate 9 according to the embodiment of Figure 6. Since these discrete capacitors 6 require more space than integrated capacitors 6 as used in the embodiment shown in Figures 4 and 5, the outer contour of the paddle structure 3 is somewhat curvier in the embodiment of Figure 6.

Consequently, the paddle structure 3 has a slightly bigger thickness in the area of the discrete capacitors 6 than in case of using integrated capacitors 6 as in the embodiments shown in Figures 4 and 5. The general functioning of the paddle structure 3 is not altered by the use of discrete capacitors 6 so that reference is made to the explanations given above with respect to the other embodiments.

The therapeutic electrode poles 4 are electrically connected with the energy dissipating electrode pole 5 only via the discrete capacitors 6. For this purpose, an electric trace 12 connects each of the therapeutic electrode poles 4 with one of the discrete capacitors 6 and the respective of the discrete capacitors 6 with the energy dissipating electrode pole 5. The electric trace 12 is fully integrated within the core substrate 9.

In the embodiment shown in Figure 6, once again all surface areas of the core substrate 9 that are not covered by either the therapeutic electrode poles 4 or the energy dissipating electrode pole 5 are covered by a silicone layer 10.

It will be apparent to those skilled in the art that numerous modifications and variations of the described examples and embodiments are possible in light of the above teaching. The disclosed examples and embodiments are presented for purposes of illustration only. Other alternate embodiments may include some or all of the features disclosed herein. Therefore, it is the intent to cover all such modifications and alternate embodiments as may come within the true scope of this invention.

## Claims

1. A paddle lead (1) for implantation in a patient's epidural space, the paddle lead (1) comprising:
an lead body (2);
a paddle structure (3) electrically coupled to a distal portion of the lead body (2) and arranged distally of the lead body (2), wherein the paddle structure (3) comprises:
a core substrate (9) made from a liquid crystal polymer;
an electrically insulating cover layer (10) surrounding the core substrate (9) at least section-wise;
a plurality of therapeutic electrode poles (4) arranged on a surface of the paddle structure (3) not covered by the electrically insulating cover layer (10);
at least one energy dissipating electrode pole (5) arranged on a surface of the paddle structure (3) not covered by the electrically insulating cover layer (10) nor by the plurality of therapeutic electrode poles (4);
at least one capacitor (6) electrically interconnecting at least one of the plurality of therapeutic electrode poles (4) with the at least one energy dissipating electrode pole (5);
wherein the plurality of therapeutic electrode poles (4) and the at least one energy dissipating electrode pole (5) are electrically interconnected with each other only by the at least one capacitor (6).

2. The paddle lead according to claim 1, **wherein** the core substrate (9) is at least partially surrounded by the electrically insulating cover layer (10), the plurality of therapeutic electrode poles (4), and the at least one energy dissipating electrode pole (5).

3. The paddle lead according to claim 1 or 2, **wherein** each of the plurality of therapeutic electrode poles (4) is electrically interconnected with the at least one energy dissipating electrode pole (5) by an individual capacitor (6).

4. The paddle lead according to any of the preceding claims, **wherein** the at least one capacitor (6) is an integrated capacitor integrated into the core substrate (9).

5. The paddle lead according to any of the preceding claims, **wherein** the at least one capacitor (6) is a discrete capacitor embedded into the core substrate (9).

6. The paddle lead according to any of the preceding claims, **wherein** the paddle structure (3) has a front side designed and arranged for facing a patient's spinal cord and a back side opposite the front side and designed and arranged for facing away from a patient's spinal cord, wherein both the plurality of therapeutic electrode poles (4) and the at least one energy dissipating electrode pole (5) are arranged on the front side.

7. The paddle lead according to claim 6, **wherein** the at least one energy dissipating electrode pole (5) is arranged in a central area between the plurality of therapeutic electrode poles (4).

8. The paddle lead according to any of the preceding claims, **wherein** the paddle structure (3) has a front side designed and arranged for facing a patient's spinal cord and a back side opposite the front side and designed and arranged for facing away from a patient's spinal cord, wherein the plurality of therapeutic electrode poles (4) is arranged on the front side and the at least one energy dissipating electrode pole (5) is arranged on the back side.

9. The paddle lead according to any of the preceding claims, **wherein** the core substrate (9) has a thickness lying in a range of from 12.5 µm to 500 µm.

10. The paddle lead according to any of the preceding claims, **wherein** the at least one capacitor (6) and the at least one energy dissipating electrode pole (5) are arranged in the same plane of the core substrate (9), wherein the at least one capacitor (6) is covered by an additional layer of liquid crystal polymer.

11. The paddle lead according to any of the preceding claims, **wherein** each of the plurality of therapeutic electrode poles (4) is electrically interconnected with the at least one capacitor (6) via one connecting wire (12) per each of the plurality of therapeutic electrode poles (4), wherein the connecting wires (12) are arranged in the same plane of the core substrate (9) as the plurality of therapeutic electrode poles (4), wherein the connecting wires (12) are covered by an additional layer of liquid crystal polymer.

12. The paddle lead according to any of the preceding claims, **wherein** the at least one capacitor (6) has a capacitance lying in range of from 100 pF to 10 nF.

13. The paddle lead according to any of the preceding claims, **wherein** only a subset of the plurality of therapeutic electrode poles (4) is electrically interconnected with the at least one energy dissipating electrode pole (5), wherein the subset consists of those therapeutic electrode poles (4) that are most prone to magnetic resonance energy heating during operation of the paddle lead in an MRI (1).

14. The paddle lead according to any of the preceding claims, **wherein** the at least one energy dissipating electrode pole (5) comprises at least one of platinum, gold, iridium, platinum iridium, iridium oxide, titanium nitride, and poly(3,4-ethylenedioxythiophene).

15. A method for manufacturing a paddle lead (1) according to any of the preceding claims, the method comprising the following steps:
providing an lead body (2);
arranging a paddle structure (3) distally of the lead body (2) and electrically coupling the paddle structure (3) to a distal portion of the lead body (2), wherein the paddle structure (3) comprises:
a core substrate (9) made from a liquid crystal polymer;
an electrically insulating cover layer (10) surrounding the core substrate (9) at least section-wise;
a plurality of therapeutic electrode poles (4) arranged on a surface of the paddle structure (3) not covered by the electrically insulating cover layer (10);
at least one energy dissipating electrode pole (5) arranged on a surface of the paddle structure (3) not covered by the electrically insulating cover layer (10) nor by the plurality of therapeutic electrode poles (4);
at least one capacitor (6) electrically interconnecting at least one of the plurality of therapeutic electrode poles (4) with the at least one energy dissipating electrode pole (5);
wherein the plurality of therapeutic electrode poles (4) and the at least one energy dissipating electrode pole (5) are electrically interconnected with each other only by the at least one capacitor (6).
